(19) Europäisches Patentamt
European Patent Office
Office européen des brevets

(11) **EP 2 618 160 A1**

(12) **EUROPÄISCHE PATENTANMELDUNG**

(43) Veröffentlichungstag:
**24.07.2013 Patentblatt 2013/30**

(51) Int Cl.:
*G01N 35/00* (2006.01)       *G01N 21/84* (2006.01)

(21) Anmeldenummer: **13163852.0**

(22) Anmeldetag: **18.02.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **18.02.2009 EP 09153113**

(62) Dokumentnummer(n) der früheren Anmeldung(en) nach Art. 76 EPÜ:
**10703910.9 / 2 399 119**

(71) Anmelder:
• **F. Hoffmann-La Roche AG**
**4070 Basel (CH)**
Benannte Vertragsstaaten:
**AT BE BG CH CY CZ DK EE ES FI FR GB GR HR HU IE IS IT LI LT LU LV MC MK MT NL NO PL PT RO SE SI SK SM TR**
• **Roche Diagnostics GmbH**
**68305 Mannheim (DE)**
Benannte Vertragsstaaten:
**DE**

(72) Erfinder:
• **Miltner, Karl**
**67227 Frankenthal (DE)**
• **LORENZ, Robert**
**67547 Worms (DE)**
• **PORSCH, Ulrich**
**69469 Weinheim (DE)**
• **Knapp, Clemens**
**58519 Viernheim (DE)**

(74) Vertreter: **Pfiz, Thomas et al**
**Patentanwälte Wolf & Lutz**
**Hauptmannsreute 93**
**70193 Stuttgart (DE)**

Bemerkungen:
Diese Anmeldung ist am 16-04-2013 als Teilanmeldung zu der unter INID-Code 62 erwähnten Anmeldung eingereicht worden.

(54) **Testverfahren und Testvorrichtung zur Untersuchung einer Körperflüssigkeit**

(57)     Die Erfindung betrifft ein Testverfahren zur Untersuchung einer Körperflüssigkeit bei dem ein Testband (14) in einem Testgerät (10) eingesetzt wird, um auf dem Testband (14) bevorratete analytische Testfelder (32) sukzessive bereitzustellen, wobei das jeweils bereitgestellte Testfeld (32) durch einen Benutzer mit der Körperflüssigkeit (52) beaufschlagt wird und mittels einer geräteseitigen Messeinheit (18) unter Erfassung von Messsignalen photometrisch abgetastet wird. Um die Messsicherheit zu erhöhen, wird vorgeschlagen, dass ein Kontrollwert aus einer zeitlichen Änderung der Messsignale ermittelt wird, und dass die Messsignale nach Maßgabe des Kontrollwerts als gültig verarbeitet oder als fehlerhaft verworfen werden.

Fig.3

EP 2 618 160 A1

## Beschreibung

Die Erfindung betrifft ein Testverfahren zur Untersuchung einer Körperflüssigkeit insbesondere zur Blutzuckerbestimmung bei dem ein Testband vorzugsweise in Form einer Bandkassette in einem Testgerät eingesetzt wird, um eine Vielzahl von auf dem Testband bevorrateten analytischen Testfeldern durch Bandtransport sukzessive bereitzustellen, wobei das jeweils bereitgestellte Testfeld durch einen Benutzer mit der Körperflüssigkeit beaufschlagt wird und mittels einer geräteseitigen Messeinheit unter Erfassung von Messsignalen photometrisch abgetastet wird. Die Erfindung betrifft weiter eine entsprechende Testvorrichtung.

Eine gattungsgemäße Testbandvorrichtung ist beispielsweise aus der EP-Anmeldung Nr. 08166955.8 der Anmelder bekannt. Dort ist eine Bandkassette mit einem Testband beschrieben, auf dem sich neben den analytischen Testfeldern auch Positioniermarken befinden, um für jeden betreffenden Bandabschnitt eine zuverlässige Positionierung in verschiedenen Funktionsstellungen zu gewährleisten.

Aus der DE 199 32 846 A1 ist ein Verfahren zur Erkennung der Fehlpositionierung eines optisch auswertbaren Teststreifens bekannt, das auf dem Vergleich zweier Messwerte aus voneinander in Einschubrichtung des Teststreifens in einem Testgerät beabstandeten Messflecken beruht. Die Verhältnisse bei Teststreifensystemen sind allerdings schon insoweit kaum mit Bandsystemen vergleichbar, als die Teststreifen einzeln in eine Geräteführung eingesetzt werden, während der Bandtransport und die Bandführung auf Seite des Verbrauchsmittels erfolgt.

Ausgehend hiervon liegt der Erfindung die Aufgabe zugrunde, die im Stand der Technik vorgeschlagenen Testverfahren und -vorrichtungen weiter zu verbessern und eine erhöhte Sicherheit gegen Fehlbedienung und Fehlmessungen zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Ein erster Aspekt der Erfindung geht von dem Gedanken aus, aus einer erwarteten Signaländerung eines für das Testergebnis relevanten Messsignals eine Fehleranalyse abzuleiten. Dementsprechend wird erfindungsgemäß vorgeschlagen, dass ein Kontrollwert aus einer zeitlichen und/oder wellenlängenabhängigen Änderung der Messsignale ermittelt wird, und dass die Messsignale in Abhängigkeit von einem vorgegebenen Schwellenwert des Kontrollwerts entweder als gültig verarbeitet oder als fehlerhaft verworfen werden. Auf diese Weise ist es möglich, verfälschende Fremdeinwirkungen auf das Messergebnis weitgehend auszuschließen. Es versteht sich, dass dabei auch mehrere potentielle Fehlerfälle parallel überprüfbar sind.

Eine auf den Umständen der Probenapplikation beruhende Fehlerdiskriminierung sieht vor, dass die Messsignale bei zwei unterschiedlichen Wellenlängen erfasst werden, und dass der Kontrollwert aus einer Signaldifferenz der wellenlängenunterschiedlichen Messsignale ermittelt wird, wobei bei verschwindender Signaldifferenz ein Fehlerfall geräteseitig erkannt und ggf. ein Fehlersignal ausgelöst wird. Dadurch lassen sich speziell auch solche Manipulationen erkennen, bei denen ein Benutzer beispielsweise mit dem Finger gegen das Testfeld andrückt, ohne jedoch eine Probe aufzutragen.

Vorteilhafterweise beruht die Signaldifferenz der wellenlängenunterschiedlichen Messsignale auf der Benetzung des bereitgestellten Testfelds mit der Körperflüssigkeit, so dass auch bei geringen Analytkonzentrationen eine zuverlässige Fehlererkennung möglich ist. In diesem Zusammenhang ist es günstig, wenn die wellenlängenunterschiedlichen Messsignale im sichtbaren Wellenlängenbereich und im Infrarotbereich gewonnen werden.

Eine weitere vorteilhafte Ausgestaltung besteht darin, dass der Kontrollwert aus einer Signaldifferenz von am Anfang und Ende eines Messintervalls erfassten Messsignalen ermittelt wird, wobei bei verschwindender Signaldifferenz ein Fehlerfall erkannt wird. Diese Art der Fehlererkennung beruht auf der speziellen Reaktionskinetik eines Analyten auf farbveränderlichen Testfeldern, die sich somit von einer mechanischen Bandmanipulation unterscheiden lässt.

Gemäß einer weiteren vorteilhaften Ausgestaltung werden die Messsignale über die Dauer eines Messintervalls erfasst, wobei der Kontrollwert aus einer Messsignaländerung in einem anfänglichen Zeitabschnitt des Messintervalls ermittelt wird, und wobei bei einer Messsignaländerung unterhalb eines vorgegebenen Mindestwerts ein Fehlerfall erkannt wird. Auf diese Weise lassen sich auch Umwelteinflüsse ausschließen, die im Vergleich zu einer regulären Messung nur zu einer deutlich reduzierten anfänglichen Signaländerung führen.

In der Bereitstellungsphase für die Flüssigkeitsapplikation ist es von Vorteil, wenn an dem bereitgestellten Testfeld zyklisch Leerwerte erfasst werden, und wenn der Kontrollwert aus einer Leerwertänderung gegenüber einem anfänglichen Leerwert ermittelt wird, wobei bei einer Leerwertänderung über dem Schwellenwert eine Flüssigkeitsapplikation und unterhalb davon ein Fehlerfall erkannt wird.

Vorteilhafterweise wird bei einer Leerwertänderung bis zu einem vorgegebenen Grenzwert der momentane Leerwert zur Bestimmung eines relativen Messwerts für einen Analyten in der Körperflüssigkeit berücksichtigt. Dadurch lässt sich ein referenzierter Messwert gewinnen, ohne dass eine geringe Änderung der Bezugsgröße zu einem verfälschten Ergebnis führt. Die vorgenannten Vorteile ergeben sich auch für eine korrespondierende Vorrichtung zur Ausführung des erfindungsgemäßen Verfahrens.

Ein weiterer Erfindungsaspekt liegt darin, dass ein Chargenkontrollwert auf einem dem Testband zugeordneten Speichermittel gespeichert wird, dass aus einer Leermessung des noch unbenutzten ersten Testfelds ein Testfeldkontrollwert ermittelt wird, und dass die Verwertbarkeit des ersten Testfelds durch Vergleich des Chargenkontrollwerts und des Testfeldkontrollwerts bestimmt wird. Durch einen solchen Qualitätscheck können schädliche Einflüsse auf das als Verbrauchsmittel beispielsweise erst nach längerer Lagerzeit eingesetzte Testmaterial erkannt werden. Als Ergebnis dieser Prüfung kann auch das gesamte Testband als nicht verwertbar eingestuft werden, wenn der Testfeldkontrollwert des ersten Testfelds auf dem Testband um mehr als eine vorgegebene Toleranz von dem Chargenkontrollwert abweicht.

Vorteilhafterweise wird der Chargenkontrollwert im Zuge einer chargenweisen Herstellung von Testbändern durch Vermessen von Testfeldern und Kalibrierfeldern auf dem Testbandmaterial bestimmt. Dies lässt sich aufgrund der homogenen Verarbeitungsprozesse bei Herstellung der Tests in Bandform zuverlässig durchführen.

Um eine tolerierbare Veränderung für nachfolgende Messungen zu berücksichtigen, ist es vorteilhaft, wenn der Testfeldkontrollwert eines bereitgestellten und als verwertbar eingestuften Testfelds des Testbands als neuer Bandkontrollwert geräteseitig gespeichert wird, und wenn zur Verwertbarkeitsprüfung des nächsten Testfelds dessen entsprechend ermittelter Testfeldkontrollwert mit dem gespeicherten Bandkontrollwert verglichen wird.

Eine vorteilhafte Messwertreferenzierung lässt sich dadurch realisieren, dass durch Erfassung eines dem jeweiligen Testfeld zugeordneten, vorzugsweise weißen Kalibrierfelds mittels der Messeinheit eine Kalibriermessung durchgeführt wird, und dass der Testfeldkontrollwert als Relativwert aus der Leermessung und der Kalibriermessung ermittelt wird.

Für eine weitgehend automatische Verarbeitung ist es auch von Vorteil, wenn der Chargenkontrollwert in einem auf der Bandkassette aufgebrachten Speichermittel, vorzugsweise in einem RFID-Chip gespeichert wird, so dass ein geräteseitiger Vergleich ohne weitere Benutzerinteraktion erfolgen kann.

Ein besonderer Aspekt der Erfindung ist auch darin zu sehen, dass ein Signaloffset der Messeinheit in einem dem bereitgestellten Testfeld zugeordneten Referenzbereich des Testbands erfasst wird, und dass bei einem einen vorgegebenen Grenzwert übersteigenden Signaloffset eine Fehlermeldung ausgelöst wird. Auf diese Weise lassen sich Verschmutzungen oder sonstige Veränderungen im optischen Strahlengang zuverlässig erkennen.

Eine weitere Verbesserung sieht vor, dass der Signaloffset an einem dunkel gefärbten Schwarzfeld als Referenzbereich des Testbands erfasst wird, wobei das Schwarzfeld auf einem dem jeweiligen Testfeld benachbarten Bandabschnitt angeordnet ist und durch Bandtransport im Erfassungsbereich der Messeinheit positioniert wird.

Im Folgenden wird die Erfindung anhand eines in der Zeichnung dargestellten Ausführungsbeispiels näher erläutert. Es zeigen:

Fig. 1 ein analytisches Testbandsystem zur Blutzuckerbestimmung umfassend ein Handgerät und eine Testbandkassette in einer aufgeschnittenen perspektivischen Darstellung;

Fig. 2 eine Ausschnittsvergrößerung der Fig. 1 im Bereich einer Messspitze;

Fig. 3 einen Testbandabschnitt in der Draufsicht;

Fig. 4 ein Messwert-Diagramm in verschiedenen Phasen des Messablaufs;

Fig. 5 ein Messwert-Diagramm in Abhängigkeit der Konzentration eines Analyten für zwei verschiedene Wellenlängen.

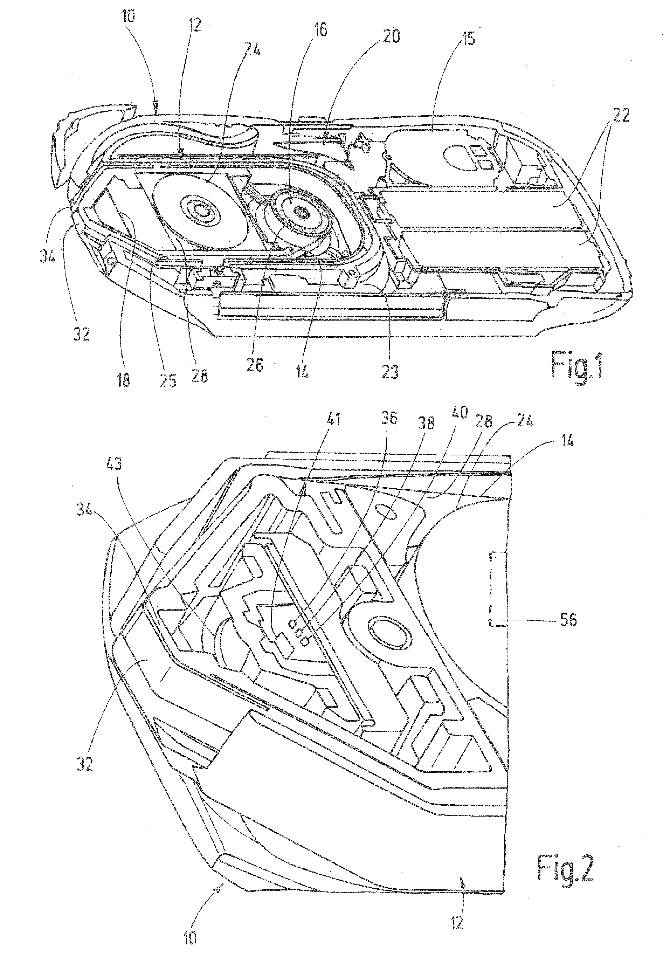

Das in Fig. 1 gezeigte Testbandsystem ermöglicht in einem Handgerät 10 den Einsatz einer Bandkassette 12 mit vorspulbarem Testband 14 als Verbrauchsmittel zur Durchführung von Glukosetests, wobei Funktionsprüfungen in verschiedenen Phasen des Messablaufs vorgenommen werden. Das generelle Geräteprinzip ist in der EP-Anmeldung Nr. 02026242.4 beschrieben, worauf hier Bezug genommen wird.

Das Handgerät 10 besitzt einen Bandantrieb (Motor 15 mit Antriebsspindel 16), eine Messeinheit 18, eine mikroprozessorgestützte Steuereinrichtung 20 und eine Energieversorgung 22. Eine nicht gezeigte Anzeige ermöglicht die Ausgabe von Messergebnissen und Gerätemeldungen für den Benutzer.

Die in ein Aufnahmefach 23 des Geräts 10 einsetzbare Bandkassette 12 umfasst eine Vorratsspule 24 für unverbrauchtes Testband 14 und eine mit dem Antrieb 16 koppelbare Aufwickelspule 26 für verbrauchtes Testband sowie eine Bandführung 25 mit einer Umlenkspitze 34. Die Vorratsspule 24 ist in einer gegenüber der Umgebung abgedichteten Vorratskammer 28 angeordnet.

Das Testband 14 ist abschnittsweise mit Testfeldern 32 versehen, die somit in einer gegebenen Reihenfolge in Bandtransportrichtung gesehen angeordnet sind. Hierbei ist zu berücksichtigen, dass die mit Blut kontaminierten Testfelder 32 auf der Aufwickelspule 26 entsorgt werden und somit ein Rückspulen des Bandes nicht praktikabel ist.

Das jeweils bereitgestellte bzw. aktive Testfeld 32 ist im Bereich der von außen zugänglichen Umlenkspitze 34 vorderseitig mit Probenflüssigkeit, insbesondere Blut oder Gewebeflüssigkeit beaufschlagbar. Der Nachweis des Analyten (Glukose) erfolgt durch rückseitige reflexionsphotometrische Erfassung einer Farbänderung der Testfelder 32 mittels der Messeinheit 18. Zu diesem Zweck sind die Testfelder 32 als Trockenreagenzschicht auf einer transparenten Trägerfolie aufgebracht. Durch entsprechenden Bandvorlauf können die Testfelder 32 sukzessive zum Einsatz gebracht werden. Auf diese Weise lassen sich Vielfachtests für eine Patienten-Selbstkontrolle durchführen, ohne dass Verbrauchsmittel häufig ausgetauscht werden müssten.

Wie in Fig. 2 gezeigt, weist die in die Kassette 12 eingreifende gerätefeste Messeinheit 18 drei Leuchtdioden 36, 38, 40 als Strahlungsquelle sowie eine Photodiode 41 als Detektor für eine reflektionsphotometrische Signalerfassung auf. Eine Optik 43 ermöglicht einen gebündelten Strahlengang unter Abbildung von Leuchtflecken definierter Größe und Intensität auf der Bandrückseite. Die mittlere Leuchtdiode 38 strahlt im sichtbaren (roten) Wellenlängenbereich bei ca. 650 nm, während die äußeren Leuchtdioden 36, 40 im Infrarot bei 875 nm arbeiten. Das auf dem Teststreifen 48 rückwärts gestreute Licht wird mit der Photodiode 41 in einem gegebenen Zeittakt erfasst.

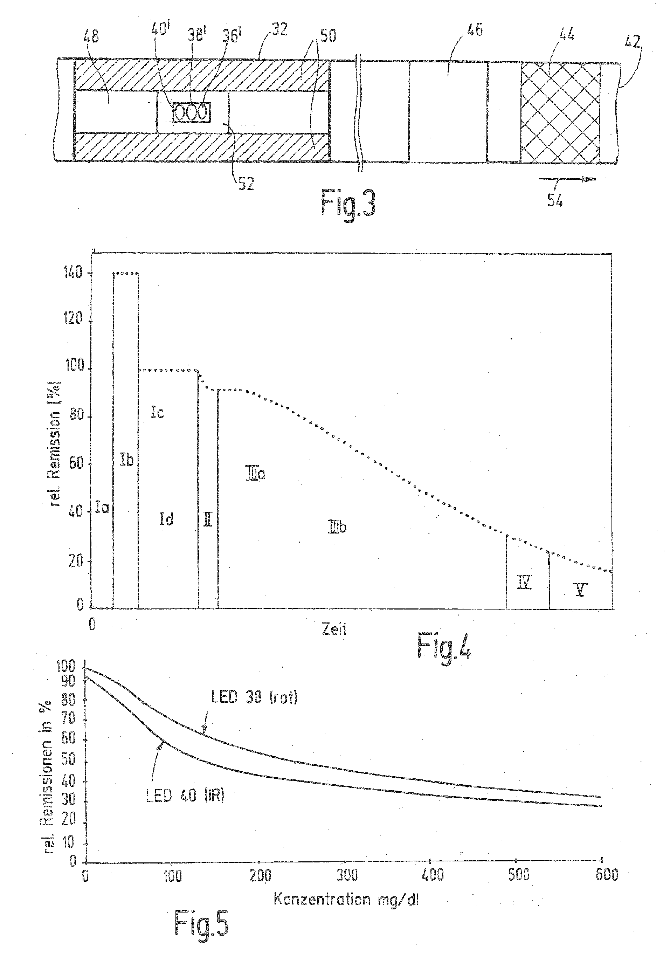

Wie in Fig. 3 veranschaulicht, befinden sich die voneinander beabstandeten Testfelder 32 einzeln auf jeweils einem zugeordneten Bandabschnitt 42, der zusätzlich mit weiteren Prüf- bzw. Kontrollfeldern in Form eines Schwarzfelds 44 und eines Weißfelds 46 versehen ist. Das Testfeld 32 weist einen durch die Testchemieschicht gebildeten zentralen Teststreifen 48 auf, der von zwei hydrophoben Randstreifen 50 seitlich begrenzt ist. Die auf dem Testfeld 32 vorderseitig applizierte Probenflüssigkeit benetzt den Teststreifen 48 in Form eines Probenflecks 52, der durch die Leuchtflecken 36', 38', 40' der Leuchtdioden 36,38,40 in der Messposition an der Umlenkspitze 34 von der transparenten Bandrückseite her abgetastet wird. Aufgrund des nur in einer Richtung (Pfeil 54) möglichen Bandtransports werden allerdings zunächst die Prüffelder 44, 46 vor der eigentlichen Messung erfasst, wie nachstehend näher erläutert.

In der Standby-Position befindet sich das Weißfeld 46 des noch unbenutzten Bandabschnitts 42 an der Umlenkspitze 34 vor der Messeinheit 18. Das in weißer Farbe auf das Trägerband 34 aufgedruckte Weißfeld 46 ist so bemessen, dass das von der Messeinheit 18 erfasste Messfenster vollständig überdeckt wird. In gleicher Weise kann auch ein vorgeordnetes Schwarzfeld 44 vor Einnahme der Standby-Position zur Vermessung positioniert werden.

Wie aus Fig. 4 ersichtlich, ist der Messzyklus für jeden Bandabschnitt 42 in verschiedene Phasen unterteilt. In der Phase Ia wird das Schwarzfeld 44 zur Verschmutzungserkennung und ggf. zur geräteseitigen Eigenkorrektur abgetastet, wie es nachstehend noch näher erläutert wird. In Phase Ib erfolgt die Vermessung des Weißfelds 46 zur Bandqualitätsüberprüfung und ggf. zur Eigenkorrektur. Phase Ic sieht die Gewinnung eines so genannten Trockenleerwerts TLW an dem noch unbenutzten Testfeld 32 vor. Sodann ergeht eine Aufforderung zum Blutauftrag an den Benutzer (Id). Die Bereitstellungsphase ist damit abgeschlossen.

In Phase II erfolgt eine Benetzungserkennung an dem Testfeld 32 mittels der IR-Leuchtdioden 36, 40. Bei der Benetzung des Teststreifens 48 findet ein Einbruch der Signalintensität statt.

Anschließend wird die Kinetik des analytspezifischen Messsignals anhand der Farbveränderung des Teststreifens 48 in den Phasen III und IV im Messtakt von z.B. 0,2 s verfolgt. Die Endphase IIIb der Kinetikverfolgung ergibt sich bei Erreichen einer Abbruchschwelle der sich in Abhängigkeit von der chemischen Reaktionsgeschwindigkeit abschwächenden Signaländerung. Sodann wird in Phase IV eine Zweifachmessung mittels LED 38 vorgenommen, um einen gemittelten Endwert EW zu ermitteln. Die Bestimmung der Glukosekonzentration erfolgt dann in relativer Remission, indem der Quotient aus diesem Endwert EW und dem Trockenleerwert TLW gebildet wird (allgemein errechnet sich die relative Remission aus dem Verhältnis des aktuellen Messwerts zum Trockenleerwert). Phase V sieht noch eine Homogenitätsmessung des Probenflecks 52 zur Unterdosiererkennung vor, die auf dem quantitativen Signalvergleich der beiden IR-LEDs 36, 40 beruht. Im Display des Geräts 10 wird schließlich die Glukosekonzentration für den Benutzer angezeigt.

Außer der eigentlichen Messung zur Bestimmung der Glukosekonzentration werden die oben angesprochenen Funktionen bzw. "Fail-Safes" folgendermaßen realisiert:

Die Verschmutzungserkennung erfolgt mit LED 38 nach dem Einlegen der Kassette 12 und im Anschluss an jede Glukosemessung über eine Messung des Signaloffsets auf dem Schwarzfeld 44. Dieser Offset wird von dem gesamten Messumfeld bei eingeschalteten LEDs erzeugt, ohne dass ein Test beteiligt ist. Er geht damit als additive Größe bei der Ermittlung des Messwerts ein. Aufgrund von Verschmutzungen oder sonstigen Optikveränderungen im Strahlengang beispielsweise durch Fremdkörper, Staub und Kratzer wird ausgesendetes Licht teilweise reflektiert und zum Detektor 41 geleitet. Das Schwarzfeld 44 dient bei der Offseterkennung als Ersatz für einen schwarzen Hohlraum, der kein Licht zurückwirft. Grundsätzlich wäre es aber auch möglich, durch das transparente Trägerband hindurch in den dunklen Geräteinnenraum hinein zu messen.

Der erfasste Signaloffset wird mit einem im Gerät 10 gespeicherten Grenzwert verglichen, der bei der Produktherstellung als chargenmittelwert ermittelt wurde. Wird der hinterlegte Grenzwert überschritten, so wird eine Fehlermeldung ausgelöst.

Um die Qualität der als Einwegartikel ggf. nach längerer Lagerzeit eingesetzten Bandkassette 12 zu überprüfen, wird zumindest an dem ersten Weißfeld 46 auf dem Testband 14 ein Referenzwert WF erfasst. Anschließend wird eine potentielle Schädigung der Teststreifenchemie beispielsweise aufgrund von Umwelteinflüssen durch eine entsprechende Veränderung des Trockenleerwerts TLW des ersten Testfelds 32 nachgewiesen. Dazu wird nicht der absolute Remissionswert herangezogen, sondern der auf den Referenzwert WF bezogene relative Remissionswert.

Ein entsprechender Chargenkontrollwert CC wird im Zuge einer der chargenweisen Herstellung von Testbändern 14 durch Vermessen von Testfeldern 32 und Weißfeldern 46 auf dem Testbandmaterial bestimmt. Die Bandherstellung erfolgt in einem Rolle-zu-Rolle-Prozess, der eine solche Kontrollwertzuordnung zu einer weitgehend einheitlichen Beschichtung erlaubt. Der Chargenkontrollwert wird in einem RFID-Chip 56 auf der Kassette 12 gespeichert und durch die Geräteelektronik 20 ausgelesen und verarbeitet. Der RFID-Chip 56 ist außenseitig auf der Kassette 12 aufgebracht und in der aufgeschnittenen Darstellung nach Fig. 2 nur symbolisch gezeigt.

Die Testfeld-Qualitätsprüfung fällt negativ aus, wenn folgende Bedingung erfüllt ist:

$$TLW_1 / WF_1 < CC - \Delta C \quad (1)$$

wobei $\Delta C$ ein Toleranzwert ist und der Index 1 sich auf den ersten Testbandabschnitt 42 bezieht. In diesem Fall wird eine entsprechende Fehlermeldung ausgegeben und die Kassette 12 ggf. verworfen.

Bei positivem Ergebnis ist es auch möglich, für die nachfolgenden Tests eine Qualitätsprüfung in engeren Schranken durchzuführen. Hierzu wird der relative Remissionswert $C_{n-1}$ des aktuell benutzten Testfelds in einem Gerätespeicher abgespeichert und entsprechend der obenstehenden Gleichung (1) anstelle des Chargenkontrollwerts CC eingesetzt. Eine nachfolgende Qualitätsprüfung eines nächsten Testfelds n fällt also negativ aus, wenn:

$$TLW_n / WF_n < C_{n-1} - \Delta C \quad (2).$$

Generell wird durch eine herstellerseitige Kalibrierung der Geräte 10 sichergestellt, dass relevante Messwerte nur im spezifizierten Messbereich aller optoelektronischen Komponenten erzeugt werden können. Hierbei erfolgt eine Kalibrierung der elektrischen Parameter der drei LEDs 36, 38, 40 und der optischen Kenngrößen.

Prinzipiell ist auch ein Eigenkorrekturverfahren bzw. eine geräteseitige Kalibrierung möglich, um den spezifischen Einfluss des Signaloffsets und schwankender Absolutmesswerte auf die Messwertbestimmung zu minimieren. Herstellungsbedingt variiert der optische Offset von Kassette zu Kassette. Hinzu kommt, dass aufgrund der Trennung von geräteseitiger Optik und kassettenseitiger Bandführung ein mit Toleranzen behafteter Abstandsanteil auftritt.

Für die Referenzmessung dienen wiederum die Schwarz- und Weißfelder 44, 46, die sich auf dem Testband 14 vor jedem Testfeld 32 befinden. Bereits in der Produktion werden diese Felder vermessen und mit einem Chargenmittelwert versehen. Diese Werte werden als Referenzwert auf dem RFID-Chip 56 abgelegt.

Der nach dem Einlegen einer Kassette 12 an dem ersten Schwarzfeld 44 gemessene Schwarzfeldwert wird überprüft, ob er mit einer vorgegebenen Toleranz am herstellerseitigen Chargenmittelwert für den optischen Offset liegt. Ist dies der Fall, wird der Chargenmittelwert beibehalten. Weicht der gemessene Schwarzfeldwert von diesem Toleranzbereich ab, so wird die Differenz zu dem Chargenmittelwert bestimmt und dem optischen Offset zugerechnet. Der optische Offset wird bei den nachfolgend an den Testfeldern 32 gewonnenen Brutto-Messsignalen subtrahiert.

Die Korrektur des optischen Offsets wird jedoch nur bis zu einem festgelegten Grenzwert durchgeführt. Bei einer Überschreitung dieser Grenze wird wie oben beschrieben eine Fehlermeldung ausgelöst. Vor jedem nachfolgenden Test wird die Schwarzfeldmessung nur zur Verschmutzungserkennung verwendet.

Bei der Weißfeldkalibrierung wird der individuelle Empfindlichkeitswert der Kassette bestimmt, indem der am Weißfeld 46 erfasste Messwert mit dem auf dem RFID-Chip 56 hinterlegten Chargenmittelwert in absoluter Remission verglichen wird. Liegt der gemessene Weißfeldwert $m_K$ innerhalb eines Toleranzbereichs am Chargenmittelwert $m_W$, so wird für eine nachfolgende Skalierung des Offset-korrigierten Brutto-Messsignals der Chargenmittelwert $m_W$, herangezogen und ansonsten die individuelle Kassettenempfindlichkeit $m_K$. Bei der Überschreitung eines Grenzwertes der Abweichung wird jedoch eine Fehlermeldung ausgegeben.

Um eine ungewollte Messwerterzeugung durch Fehlbedienung weitgehend auszuschließen, kann ein Kontrollwert aus einer zeitlichen und/oder wellenlängenabhängigen Änderung der Messsignale ermittelt werden, wobei die Messsignale dann in Abhängigkeit von einem Schwellenwer des Kontrollwerts als gültig weiter verarbeitet oder als fehlerhaft verworfen werden.

Ein erster solcher Fehlerfall kann darin bestehen, dass aufgrund der Abstandsabhängigkeit des Messprinzips durch Andrücken gegen die Umlenkspitze 34 ein Messstart und ein artifizielles Messergebnis erzeugt werden könnte, ohne dass eine Probe appliziert wurde. Um dies ausschließen zu können, werden Messsignale an dem Testfeld 32 bei zwei unterschiedlichen Wellenlängen erfasst, wobei der Kontrollwert aus einer Signaldifferenz der wellenlängenunterschiedlichen Messsignale ermittelt wird.

Wie aus Fig. 5 ersichtlich, ergeben sich bei einer Probenvermessung mit unterschiedlichen Wellenlängen unterschiedliche Werte der relativen Remission über den gesamten Bereich der zu untersuchenden Analyt- bzw. Glukosekonzentration. Dieser Signalunterschied entsteht durch Befeuchten des Testfelds 32 mit der Körperflüssigkeit (deshalb ist auch bei Probenkonzentration 0 bereits ein Unterschied zu finden) und verstärkt sich, wenn das Testchemiesystem verstärkt Reaktionsfarbe bildet. Wird also nur durch Andrücken ein Messsignal erzeugt, kann der typische Unterschied in den beiden wellenlängenunterschiedlichen LEDs 38, 40 durch das fehlende Befeuchten und die fehlende Reaktionsfarbe nicht beobachtet werden, wodurch ein Fehlerfall erkannt wird. Der vorgegebene Schwellenwert des Signalunterschieds kann beispielsweise bei 3% relativer Remission liegen.

Ein weiteres Szenario einer ungewollten Messwerterzeugung besteht darin, dass die fälschliche Erkennung eines Blutauftrags durch eine Bandverschiebung provoziert wird. Wird durch eine Benutzermanipulation ein dunkler Randstreifen 50 des Testfelds 32 in den Strahlengang der Messeinheit 18 verschoben, können hohe Messwerte erzeugt werden, ohne dass Probe aufgetragen wurde.

Typische Reaktionskinetiken von Blutproben auf einem Testfeld 32 zeigen jedoch oberhalb von ca. 100 mg/dl Glukosekonzentration einen Signalhub von ca. 10%, wie er als Differenz der ersten und letzten Kinetikmessung in Phase III (Fig. 4) ermittelt wird. Wird hingegen das Testband in Phase II wie oben beschrieben bloß verschoben, erfolgt ein schlagartiges Verdunkeln und danach ein konstantes Signal - es wird also in Phase III keine veränderliche Reaktionskinetik und kein nennenswerter Signalhub beobachtet. Somit ist eine Fehlererkennung dadurch möglich, dass der Kontrollwert aus einer Signaldifferenz von am Anfang und Ende eines Messintervalls erfassten Messsignalen ermittelt wird, und dass bei einer Signaldifferenz nahe Null ein Fehlerfall erkannt wird.

Befindet sich ein Testfeld 32 vor der Optik 43 im Zustand der Probenauftragserkennung (Phase II in Fig. 4), so interpretiert die Steuereinrichtung 20 des Geräts 10 eine Signaländerung um ein festgelegtes Maß als Probenauftrag und beginnt mit der Auswertung. Sowohl hohe Luftfeuchtigkeit als auch Sonneneinstrahlung können unter ungünstigen Umständen ohne Probenauftrag bereits zu einer solchen Signaländerung und damit zu einem Messstart führen.

Um dies zu verhindern, wird der zeitliche Verlauf der Leersignaländerung im Zustand der Probenerwartung geprüft. Während das Auftragen einer Blutprobe bereits innerhalb einer halben Sekunde einen Remissionsabfall von mehreren Prozent erzeugt, wird ein solcher Abfall bei Einwirkung von Sonnenlicht oder Luftfeuchte erst über einen Zeitraum von mehr als 20 Sekunden erreicht. Demzufolge ist es möglich, dass an dem für die Probenapplikation bereitgestellten Testfeld zyklisch Leerwerte erfasst werden, und dass der Kontrollwert aus einer Leerwertänderung gegenüber einem anfänglichen Leerwert ermittelt wird, wobei bei einer Leerwertänderung über einem vorgegebenen Schwellenwert (von beispielsweise ca. 5%) eine Flüssigkeitsapplikation und unterhalb davon ggf. nach einer gegebenen Wartezeit ein Fehlerfall erkannt wird.

Ein weiteres Messproblem kann darin bestehen, das sich der Trockenleerwert eines bereitgestellten, aber noch unbenutzten Testfelds 32 beispielsweise durch Licht- oder Feuchtigkeitseinfluss verändert und damit als Bezugsgröße für die Bestimmung der relativen Remission zu einer Verfälschung führt. Der Messwert des unbenutzten Testfelds kann daher im Zustand der Probenerwartung zyklisch überprüft und entweder nachgeführt werden, um somit Messwertverfälschungen zu verhindern, oder ab einem bestimmten Grenzwert beispielsweise von mehr als 0,5%/s relativer Remissionsänderung die Messung mit einer Fehlermeldung abzubrechen.

## Patentansprüche

1. Testverfahren zur Untersuchung einer Körperflüssigkeit insbesondere zur Blutzuckerbestimmung bei dem ein Testband (14) vorzugsweise in Form einer Bandkassette (12) in einem Testgerät (10) eingesetzt wird, um eine Vielzahl von auf dem Testband (14) bevorrateten analytischen Testfeldern (32) durch Bandtransport sukzessive bereitzustellen, wobei das jeweils bereitgestellte Testfeld (32) durch einen Benutzer mit der Körperflüssigkeit (52) beaufschlagt wird und mittels einer geräteseitigen Messeinheit (18) unter Erfassung von Messsignalen photometrisch abgetastet wird, **dadurch gekennzeichnet, dass** ein Kontrollwert aus einer zeitlichen Änderung der Messsignale ermittelt wird, und dass die Messsignale bei Unterschreiten eines vorgegebenen Schwellenwerts des Kontrollwerts als fehlerhaft verworfen werden.

2. Testverfahren nach Anspruch 1, **dadurch gekennzeichnet, dass** der Kontrollwert aus einer Signaldifferenz von am Anfang und Ende eines Messintervalls erfassten Messsignalen ermittelt wird.

3. Testverfahren nach Anspruch 1 oder 2, **dadurch gekennzeichnet, dass** die Messsignale über die Dauer eines Messintervalls erfasst werden, und dass der Kontrollwert aus einer Messsignaländerung in einem anfänglichen Zeitabschnitt des Messintervalls ermittelt wird.

4. Testverfahren nach einem der Ansprüche 1 bis 3, **dadurch gekennzeichnet, dass** an dem für die Flüssigkeitsapplikation bereitgestellten Testfeld (32) zyklisch Leerwerte erfasst werden.

5. Testverfahren nach Anspruch 4, **dadurch gekennzeichnet, dass** der Kontrollwert aus einer Leerwertänderung gegenüber einem anfänglichen Leerwert ermittelt wird,

6. Testverfahren nach Anspruch 4 oder 5, **dadurch gekennzeichnet, dass** bei einer Leerwertänderung über dem Schwellenwert das Vorhandensein einer Körperflüssigkeit erkannt wird.

7. Testverfahren nach einem der Ansprüche 4 bis 6, **dadurch gekennzeichnet, dass** bei einer Leerwertänderung bis zu einem vorgegebenen Grenzwert der momentane Leerwert zur Bestimmung eines relativen Messwerts für einen Analyten in der Körperflüssigkeit berücksichtigt wird.

8. Testvorrichtung zur Untersuchung einer Körperflüssigkeit insbesondere zur Blutzuckerbestimmung mit einem Testgerät (10) und einem darin vorzugsweise in Form einer Bandkassette (12) eingesetzten Testband (14), welches eine Vielzahl von analytischen Testfeldern (32) jeweils in einem zugeordneten Bandabschnitt (42) aufweist, wobei die Testfelder (32) durch Bandtransport sukzessive zum Auftragen von Körperflüssigkeit (52) bereitstellbar und mittels einer geräteseitigen Messeinheit (18) unter Erfassung von Messsignalen abtastbar sind, **dadurch gekennzeichnet, dass** das Testgerät (10) eine Steuereinrichtung (20) aufweist, die dazu ausgebildet ist, einen Kontrollwert aus einer zeitlichen Änderung der Messsignale zu ermitteln und die Messsignale nach Maßgabe des Kontrollwerts als gültig zu verarbeiten oder als fehlerhaft zu verwerfen.

9. Testvorrichtung nach Anspruch 8, **dadurch gekennzeichnet, dass** die Steuereinrichtung (20) die Messsignale über die Dauer eines Messintervalls erfasst und den Kontrollwert aus einer Messsignaländerung in einem anfänglichen Zeitabschnitt des Messintervalls ermittelt.

Fig.1

Fig.2

Fig.3

Fig.4

Fig.5

Europäisches
Patentamt

European
Patent Office

Office européen
des brevets

# EUROPÄISCHER RECHERCHENBERICHT

Nummer der Anmeldung

EP 13 16 3852

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 051 901 A (ENDO HIROTOSHI [JP]) 24. September 1991 (1991-09-24) | 1,2,8 | INV. G01N35/00 |
| Y | * Spalte 1, Zeile 63 - Spalte 2, Zeile 18 * | 3-7,9 | G01N21/84 |
| | * Spalte 2, Zeile 31 - Zeile 36 * * Spalte 5, Zeile 29 - Spalte 6, Zeile 32 * | | |
| | * Spalte 4, Zeile 15 - Zeile 27 * * Spalte 9, Zeile 5 - Zeile 40 * * Spalte 11, Zeile 15 - Zeile 18 * * Anspruch 2 * ----- | | |
| Y | US 5 304 468 A (PHILLIPS ROGER [US] ET AL) 19. April 1994 (1994-04-19) * Spalte 4, Zeile 8 - Zeile 22 * * Spalte 9, Zeile 11 - Zeile 36 * * Spalte 16, Zeile 15 - Zeile 36 * ----- | 4-7 | |
| Y | WO 99/18426 A1 (77 ELEKTRONIKA MUSZERIPARI KFT [HU]; TAJNAFOI GABOR [HU]) 15. April 1999 (1999-04-15) * Seite 15, Zeile 14 - Zeile 22 * ----- | 3-6,9 | |
| A | EP 1 785 730 A1 (HOFFMANN LA ROCHE [CH]; ROCHE DIAGNOSTICS GMBH [DE]) 16. Mai 2007 (2007-05-16) * Zusammenfassung * * Absatz [0022] - Absatz [0025] * ----- | 1-9 | RECHERCHIERTE SACHGEBIETE (IPC) G01N |
| A | US 3 526 480 A (FINDL EUGENE ET AL) 1. September 1970 (1970-09-01) * Abbildung 1 * * Zusammenfassung * * Spalte 4, Zeile 16 - Zeile 31 * ----- | 1-9 | |

Der vorliegende Recherchenbericht wurde für alle Patentansprüche erstellt

| Recherchenort | Abschlußdatum der Recherche | Prüfer |
|---|---|---|
| München | 27. Mai 2013 | D'Alessandro, Davide |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 16 3852

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-2013

| Im Recherchenbericht angeführtes Patentdokument | | Datum der Veröffentlichung | Mitglied(er) der Patentfamilie | | | Datum der Veröffentlichung |
|---|---|---|---|---|---|---|
| US 5051901 | A | 24-09-1991 | JP | H0377049 | A | 02-04-1991 |
| | | | JP | H0810193 | B2 | 31-01-1996 |
| | | | US | 5051901 | A | 24-09-1991 |
| US 5304468 | A | 19-04-1994 | AT | 172499 | T | 15-11-1998 |
| | | | AT | 172538 | T | 15-11-1998 |
| | | | AT | 174687 | T | 15-01-1999 |
| | | | AT | 199982 | T | 15-04-2001 |
| | | | AT | 300620 | T | 15-08-2005 |
| | | | AU | 603821 | B2 | 29-11-1990 |
| | | | AU | 7675887 | A | 18-02-1988 |
| | | | CA | 1301604 | C | 26-05-1992 |
| | | | CN | 1116307 | A | 07-02-1996 |
| | | | CN | 87106256 | A | 23-03-1988 |
| | | | DE | 3752229 | D1 | 26-11-1998 |
| | | | DE | 3752229 | T2 | 02-06-1999 |
| | | | DE | 3752230 | D1 | 26-11-1998 |
| | | | DE | 3752230 | T2 | 06-05-1999 |
| | | | DE | 3752241 | D1 | 28-01-1999 |
| | | | DE | 3752241 | T2 | 17-06-1999 |
| | | | DE | 3752328 | D1 | 26-04-2001 |
| | | | DE | 3752328 | T2 | 05-07-2001 |
| | | | DE | 3752386 | D1 | 01-09-2005 |
| | | | DE | 3752386 | T2 | 24-05-2006 |
| | | | DE | 3787851 | D1 | 25-11-1993 |
| | | | DE | 3787851 | T2 | 21-04-1994 |
| | | | DK | 91594 | A | 05-08-1994 |
| | | | DK | 157092 | A | 29-12-1992 |
| | | | DK | 157192 | A | 29-12-1992 |
| | | | DK | 419187 | A | 14-02-1988 |
| | | | EP | 0256806 | A2 | 24-02-1988 |
| | | | EP | 0473241 | A2 | 04-03-1992 |
| | | | EP | 0479394 | A2 | 08-04-1992 |
| | | | EP | 0656423 | A1 | 07-06-1995 |
| | | | EP | 0816849 | A2 | 07-01-1998 |
| | | | EP | 0960946 | A2 | 01-12-1999 |
| | | | ES | 2046985 | T3 | 16-02-1994 |
| | | | ES | 2121766 | T3 | 16-12-1998 |
| | | | ES | 2124490 | T3 | 01-02-1999 |
| | | | ES | 2126562 | T3 | 01-04-1999 |
| | | | ES | 2155224 | T3 | 01-05-2001 |
| | | | ES | 2246558 | T3 | 16-02-2006 |
| | | | FI | 873356 | A | 14-02-1988 |
| | | | GR | 3026514 | T3 | 31-07-1998 |
| | | | IE | 64442 | B1 | 09-08-1995 |
| | | | IE | 950182 | L | 13-02-1988 |

EPO FORM P0461

Für nähere Einzelheiten zu diesem Anhang : siehe Amtsblatt des Europäischen Patentamts, Nr.12/82

**EP 2 618 160 A1**

## ANHANG ZUM EUROPÄISCHEN RECHERCHENBERICHT
## ÜBER DIE EUROPÄISCHE PATENTANMELDUNG NR.

EP 13 16 3852

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-2013

| Im Recherchenbericht angeführtes Patentdokument | Datum der Veröffentlichung | Mitglied(er) der Patentfamilie | Datum der Veröffentlichung |
|---|---|---|---|
| | | IE 20020900 A1 | 23-07-2003 |
| | | IE 20020901 A1 | 23-07-2003 |
| | | JP 2589053 B2 | 12-03-1997 |
| | | JP H0767698 A | 14-03-1995 |
| | | JP H0820364 B2 | 04-03-1996 |
| | | JP S63101757 A | 06-05-1988 |
| | | NO 873372 A | 15-02-1988 |
| | | NO 923399 A | 15-02-1988 |
| | | SG 63588 A1 | 30-03-1999 |
| | | SG 83640 A1 | 16-10-2001 |
| | | US 4935346 A | 19-06-1990 |
| | | US 5179005 A | 12-01-1993 |
| | | US 5304468 A | 19-04-1994 |
| | | US 5426032 A | 20-06-1995 |
| | | US 5563042 A | 08-10-1996 |
| | | US 5843692 A | 01-12-1998 |
| | | US 5968760 A | 19-10-1999 |
| | | US 6268162 B1 | 31-07-2001 |
| | | US 2001019831 A1 | 06-09-2001 |
| | | US 2003054427 A1 | 20-03-2003 |
| | | US 2003073151 A1 | 17-04-2003 |
| | | US 2003073152 A1 | 17-04-2003 |
| | | US 2003073153 A1 | 17-04-2003 |
| WO 9918426 A1 | 15-04-1999 | AU 9553998 A | 27-04-1999 |
| | | EP 1019706 A1 | 19-07-2000 |
| | | HU 9701607 A2 | 28-07-1999 |
| | | US 6448067 B1 | 10-09-2002 |
| | | WO 9918426 A1 | 15-04-1999 |
| EP 1785730 A1 | 16-05-2007 | CA 2567675 A1 | 15-05-2007 |
| | | CN 1967251 A | 23-05-2007 |
| | | EP 1785730 A1 | 16-05-2007 |
| | | ES 2402770 T3 | 08-05-2013 |
| | | JP 4713443 B2 | 29-06-2011 |
| | | JP 2007139770 A | 07-06-2007 |
| | | US 2007217950 A1 | 20-09-2007 |
| US 3526480 A | 01-09-1970 | AU 452281 B2 | 12-08-1974 |
| | | AU 5037569 A | 13-08-1970 |
| | | BE 728248 A | 11-08-1969 |
| | | CH 501922 A | 15-01-1971 |
| | | DE 1673340 A1 | 24-02-1972 |
| | | DE 1798423 A1 | 02-08-1973 |
| | | FR 1584397 A | 19-12-1969 |
| | | GB 1218745 A | 13-01-1971 |

Für nähere Einzelheiten zu diesem Anhang : siehe Amtsblatt des Europäischen Patentamts, Nr.12/82

**ANHANG ZUM EUROPÄISCHEN RECHERCHENBERICHT**
**ÜBER DIE EUROPÄISCHE PATENTANMELDUNG NR.**                    EP 13 16 3852

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-2013

| Im Recherchenbericht angeführtes Patentdokument | Datum der Veröffentlichung | Mitglied(er) der Patentfamilie | | Datum der Veröffentlichung |
|---|---|---|---|---|
| | | GB | 1218749 A | 13-01-1971 |
| | | NL | 6902107 A | 13-08-1970 |
| | | US | 3526480 A | 01-09-1970 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 08166955 A
- DE 19932846 A1

- EP 02026242 A